# DEMANDE DE BREVET EUROPEEN

(11) **EP 1 224 915 A1**
(43) Date de publication de la demande: **24.07.2002**
(21) Numéro de dépôt: 02290142.5
(22) Date de dépôt: 21.01.2002
(51) Int. Cl.: A61B 17/70

(54) **Système de réglage en position pour instrument de chirurgie rachidienne**

(30) Priorité: 23.01.2001 FR 0100873
(71) Demandeur: Stryker Spine, 33610 Cestas (FR)
(72) Inventeur: Vincent-Prestigiacomo, Philippe, 33680 Lacanau Ocean (FR)
(74) Mandataire: Le Forestier, Eric

(57) **Abrégé**

Le système de réglage en position pour un instrument de chirurgie, notamment pour la colonne vertébrale, comportant un organe allongé (2) apte à être relié à un premier élément de l'instrument, un corps (3) apte à être fixé à l'organe longitudinal et relié à un deuxième élément de l'instrument, et des moyens de liaison (8,10) de l'organe allongé (2) au corps(3) agencés pour avoir un état déverrouillé dans lequel ils autorisent un coulissement (F') de l'organe allongé (2) par rapport au corps (3) et un état verrouillé dans lequel ils interdisent le coulissement de l'organe allongé par rapport au corps.

## Description

L'invention concerne un système de réglage pour une instrumentation de chirurgie destinée à la colonne vertébrale.

Dans le cas de traumas, la fracture simple ou multiple d'une ou plusieurs vertèbres de la colonne peut avoir différentes configurations et nécessiter des mouvements de correction pour retrouver la morphologie originelle du rachis, notamment en ce qui concerne les courbures de lordose et de cyphose. Pour cela, on utilise des instruments particuliers qui permettent une telle restauration en coopération avec des systèmes d'ostéosynthèse connus par ailleurs. Le document DE G-91-12 466.2 décrit une telle instrumentation comportant un système de réglage qui comprend une tige filetée présentant deux filets opposés situés de part et d'autre d'un moyen de mise en oeuvre. Chacun des filets vient en prise avec un alésage fileté d'un corps relié à un élément de l'instrumentation. La mise en oeuvre d'un tel système est longue du fait du pas des filetages, d'une part, et, d'autre part, du fait que lors de la manipulation, les gants viennent au contact des filets qui présentent des angles vifs aptes à accrocher et à déchirer lesdits gants, ce qui nécessite des précautions supplémentaires. Cela a pour conséquence un allongement du temps opératoire très préjudiciable pour le patient.

Un but de l'invention est de fournir un système de réglage destiné à une instrumentation de chirurgie rachidienne qui soit précis et rapide à mettre en place.

Pour cela, on prévoit, selon l'invention, un système de réglage en position pour un instrument de chirurgie, notamment pour la colonne vertébrale, comportant un organe allongé apte à être relié à un premier élément de l'instrument, un corps apte à être fixé à l'organe longitudinal et relié à un deuxième élément de l'instrument, et des moyens de liaison de l'organe allongé au corps agencés pour avoir un état déverrouillé dans lequel ils autorisent un coulissement de l'organe allongé par rapport au corps et un état verrouillé dans lequel ils interdisent le coulissement de l'organe allongé par rapport au corps.

Ainsi, l'état déverrouillé permet un réglage de la position simple, précis et rapide, alors que l'état verrouillé permet de bloquer la position choisie rapidement. De ce fait, la mise en oeuvre d'un tel système est simple, précise et rapide.

Avantageusement, les moyens de liaison sont agencés pour interdire le coulissement de l'organe allongé par rapport au corps par effet de coin.

Avantageusement, les moyens de liaison comprennent au moins un élément déformable de manière élastique.

Avantageusement, l'élément déformable est apte à réaliser l'effet de coin.

Avantageusement, le système est agencé de sorte que, dans l'état verrouillé, le coulissement est verrouillé selon un seul sens.

Avantageusement, l'élément déformable présentant une surface d'appui, l'organe allongé présente une face de contact venant en contact avec la surface d'appui, lors de l'état verrouillé

Ainsi, l'organe longitudinal entraîne l'élément déformable vers la position de blocage du fait du contact entre la face de contact et la surface d'appui sans intervention de l'opérateur sur l'élément déformable. L'opérateur n'intervenant pas sur l'élément déformable, ses mains ne sont pas en contact avec des angles saillants. Ainsi, il ne risque pas d'abîmer ou de déchirer ses gants lors de l'opération.

Avantageusement, la face de contact est lisse, le blocage s'effectuant par friction.

Avantageusement, la face de contact est moletée.

Avantageusement, la surface d'appui comporte un moletage.

Avantageusement, la face de contact présente des creux, notamment des rainures.

Avantageusement, la surface d'appui comporte un bossage s'étendant en saillie apte à être reçu dans les creux.

Avantageusement, l'élément déformable est fendu sur toute ou partie de sa longueur.

Avantageusement, l'élément déformable comporte des languettes déformables à une extrémité.

Avantageusement, l'élément déformable comporte une extrémité de mise en oeuvre de déverrouillage.

Avantageusement, l'élément déformable est de forme conique.

Avantageusement, l'extrémité de mise en oeuvre compote un rebord d'appui.

Avantageusement, les moyens de liaison comprennent un second élément déformable de manière élastique.

Avantageusement, le second élément déformable est apte à venir interdire le coulissement selon l'autre sens de déplacement.

Avantageusement, le second élément déformable est le symétrique en miroir du premier élément déformable.

Avantageusement, le corps comporte au moins deux parties.

Avantageusement, le corps comporte un moyen de liaison des deux parties, notamment une bague.

On prévoit aussi, selon l'invention, un instrument de chirurgie orthopédique, notamment pour la colonne vertébrale, comportant un système de réglage en position présentant au moins une des caractéristiques ci-dessus.

D'autres caractéristiques et avantages de l'invention apparaîtront dans la description ci-après d'un mode de réalisation. Aux dessins annexés :
- la figure 1 est une vue en coupe de l'ensemble d'un mode de réalisation de l'invention ;
- la figure 2 est une vue en trois dimensions éclatée du mode de réalisation de la figure 1, sans la tige ;
- la figure 3a est une vue en trois dimensions de l'élément déformable de l'invention de la figure 1 ;
- la figure 3b est une vue en coupe selon III-III de l'élément déformable de la figure 3a ;
- la figure 4a est une vue en trois dimensions d'une partie du corps de l'invention de la figure 1 ;
- la figure 4b est une vue en coupe selon IV-IV de la partie du corps de la figure 4a ;
- les figures 5 et 6 sont des vues en coupe du mode de réalisation de la figure 1 lors de son fonctionnement ; et
- la figure 7 est une vue schématique de l'utilisation en per-op du mode de réalisation de la figure 1.

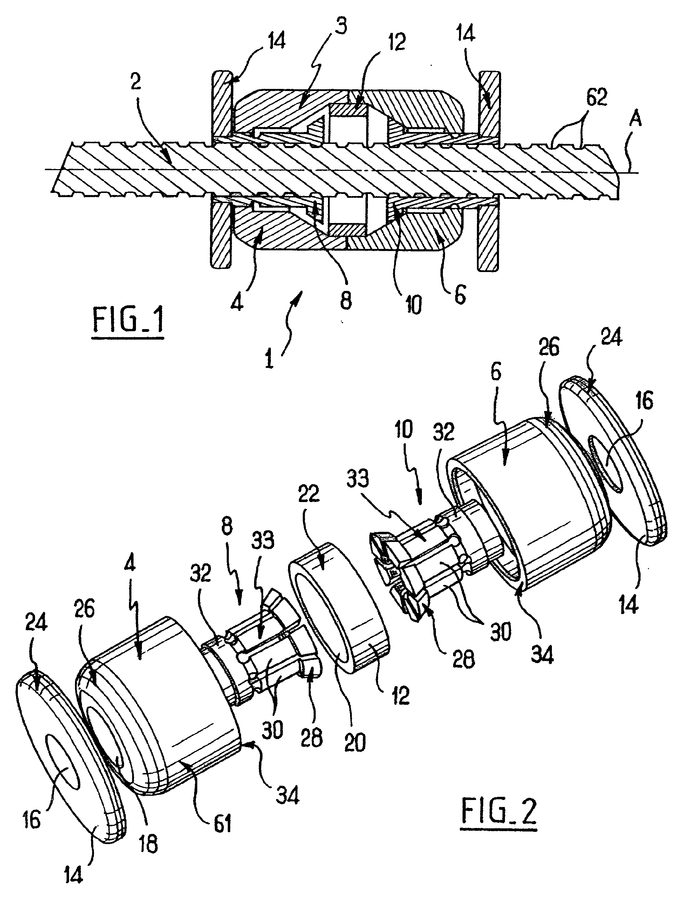

En référence à ces figures, nous allons décrire un mode de réalisation de l'invention. Sur la figure 1, le système de réglage en position 1 comporte un corps 3 creux apte à recevoir à coulissement une tige de section circulaire 2, ainsi qu'une paire d'éléments déformables élastiquement 8 et 10, à une extrémité desquels est fixé un plateau circulaire 14.

Le corps creux 3 comprend deux demi-coques 4 et 6 reliées entre elles par une bague 12. La demi-coque 6 est la symétrique en miroir de la demi coque 4 selon un plan perpendiculaire à l'axe de révolution A du corps 3. On se bornera donc à décrire en détail une demi-coque.

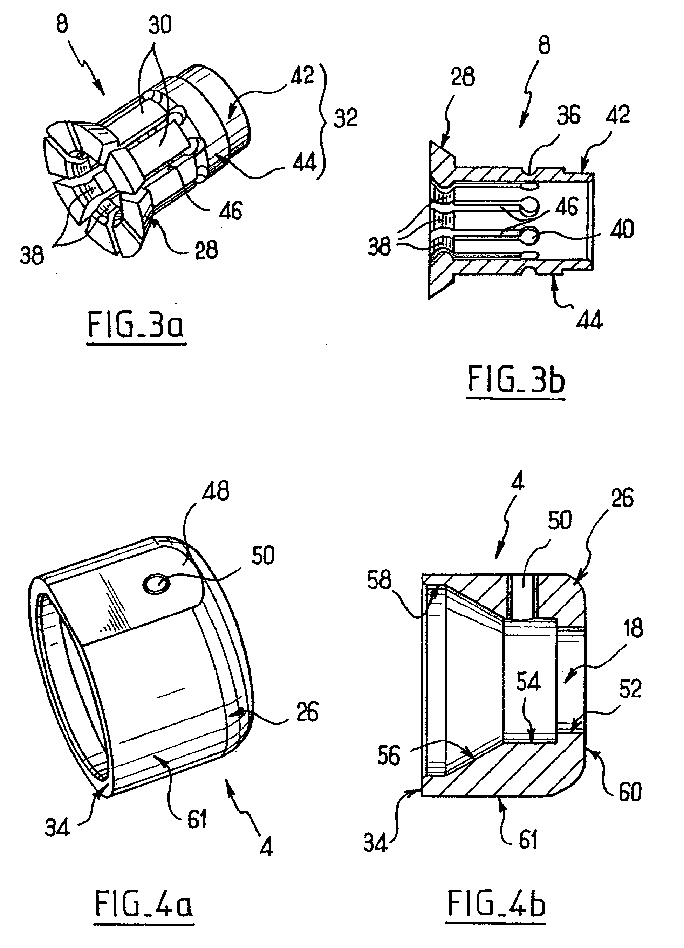

En référence aux figures 2, 4a et 4b, la demi-coque 4 présente un orifice traversant 18 s'étendant le long de son axe de révolution A. La demi-coque 4 présente une surface externe 61 ainsi que deux surfaces d'extrémité axiale 34 et 60. La surface d'extrémité 60 rejoint la surface externe 61 par une surface de forme arrondie 26. Une partie de la surface externe 61 est remplacée par un méplat 48 parallèle à l'axe de révolution A. Perpendiculaire à ce méplat 48, un orifice fileté traversant radial 50 débouche à l'intérieur de l'orifice 18. Cet orifice 18 est constitué de plusieurs parties : une première partie cylindrique de section circulaire délimitée par la surface 52 présente un premier diamètre, puis une seconde partie cylindrique de section circulaire délimitée par une surface 54 présentant un deuxième diamètre supérieur au diamètre la première partie. Dans cette partie, débouche l'orifice fileté 50. Une troisième partie en forme de surface conique 56 poursuit l'orifice 18, le faisant passer du diamètre de la seconde partie au diamètre de la quatrième partie, elle-même délimitée par la surface cylindrique 58 de section circulaire. On verra un peu plus loin que la surface 56 forme une surface d'appui.

La bague 12 servant à relier des deux demi-coques symétriques 4 et 6 présente une face cylindrique externe 22 dont le diamètre est sensiblement équivalent au diamètre de la quatrième partie de chacune des demi coques. La bague 12 présente aussi une surface cylindrique interne 20. La longueur de la bague 12 est équivalente au double de la largeur de la quatrième partie de chacune des demi-coques 4 et 6.

Le corps 3 est formé de la manière suivante : la bague 12 est insérée dans la quatrième partie de l'orifice 18 de la première demi-coque. Du fait de sa double largeur, la bague 12, une fois mise en place dans la première demi-coque, s'étend en saillie de la face 34 de la demi-coque considérée. Ensuite, la deuxième demi-coque est insérée sur la bague 12 jusqu'à ce que sa face 34 vienne en appui sur la face 34 de la première demi-coque. Ainsi, le corps 3 est entièrement formé. Les diamètres externe de la bague 12 et interne de la quatrième partie de chacune des demi coques sont choisis de manière à ce que la bague soit entrée en force pour pouvoir maintenir la cohésion du corps 3 lors de l'utilisation en intervention chirurgicale, tout en restant démontable par une instrumentiste pour le nettoyage complet de l'instrument entre deux opérations. Eventuellement, pour sécuriser le montage, un élément de liaison (non représenté) peut être utilisé et le fixant au moyen d'organes filetés (non représentés) aptes à venir en prise avec les filetages des orifices 50 de chacune des demi-coques 4 et 6, l'élément de liaison étant apte à prendre appui sur les méplats 48.

En référence aux figures 2, 3a et 3b, nous allons maintenant décrire les éléments déformables 8 et 10. L'élément 10 est le symétrique en miroir de l'élément 8 selon un plan perpendiculaire à l'axe de révolution desdits éléments A. Nous n'allons donc décrire qu'un seul des deux éléments. L'élément déformable élastiquement 8 est constitué de deux parties principales 32 et 33. L'élément déformable 8 a la forme générale d'un tube. La partie 32 présente une première surface cylindrique 42 présentant un premier diamètre et une seconde surface 44 présentant un second diamètre légèrement supérieur au diamètre de la surface 42. La partie 32 est séparée de la partie 33 de l'élément déformable 8 par une rainure concave 36 faisant le tour complet de l'élément déformable 8 et inscrite dans uns plan perpendiculaire à son axe. La partie 33 de l'élément déformable 8 est constituée d'une pluralité de languettes 30 uniformément réparties sur toute la circonférence de l'élément 8. Ici, les languettes 30 sont au nombre de 8. Chaque languette 30 est séparée de sa suivante par une fente 46 s'étendant depuis la rainure 36 jusqu'à l'extrémité axiale libre de la partie 33 de l'élément 8. Au niveau de la rainure 36, chaque fente 46 devient un orifice traversant circulaire 40. Au niveau de l'extrémité libre de la partie 33 de l'élément déformable 8, chaque languette 30 se termine par un bossage s'étendant radialement en saillie vers l'extérieur de l'élément 8 et présentant une surface 28 de forme conique. A l'opposé de cette surface 28, chacune des languettes, toujours au niveau de l'extrémité libre, présente un bossage convexe, de préférence de section circulaire, qui s'étend radialement en saillie vers l'intérieur de l'élément déformable 8. Comme nous le verrons plus loin, la partie 33 est la partie de l'élément 8 qui se déforme lors de son utilisation. Cette déformation est due à chaque languette qui est apte à se déformer au niveau de la rainure 36 essentiellement.

Le diamètre de la surface 44 est sensiblement équivalent au diamètre de la première partie de l'orifice 18 délimité par la surface 52. Le diamètre de la surface 42 est sensiblement équivalent au diamètre de l'orifice 16 présent sur les plateaux 14 que nous allons maintenant décrire brièvement.

Les plateaux 14 sont des bagues présentant un orifice 16 traversant de part en part ainsi qu'un rebord externe 24 arrondi. Les plateaux 14 sont aptes à être montés sur la partie 32, au niveau de la surface 42, de chacun des éléments déformables 8, 10.

En référence à la figure 1, l'organe allongé 2 apte à être reçu à l'intérieur du corps 3 est une tige de section circulaire et d'axe de révolution A présentant une pluralité de rainures circonférentielles 62 uniformément réparties sur toute sa longueur. Ces rainures 52 sont de section circulaire.

Nous allons maintenant aborder le montage du système de réglage en position 1. Dans la demi-coque 4 par exemple, on insère le premier élément déformable 8 de manière à ce que la surface 44 se positionne au niveau de la première partie de l'orifice 18, en contact avec la surface 52. Ensuite, un premier plateau 14 est monté en force sur la partie 32 du premier élément déformable 8 de manière à ce que l'orifice 16 du plateau 14 reçoit la surface 42 de l'élément déformable. Puis on insère en force la bague 12 dans la quatrième partie de l'orifice 18 de la demi-coque de manière à ce que la surface 22 soit en contact avec la surface 58. Ensuite, dans la deuxième demi-coque 6, on insère le deuxième élément déformable 10 de la même manière qu'on a inséré l'élément déformable 8 dans la première demi-coque 4, puis on met en place toujours de la même manière le deuxième plateau 14 sur la partie 32 de l'élément déformable 10. Enfin, la deuxième demi-coque 6 est insérée sur la bague 12 pour refermer l'ensemble du corps 3. Il est à noter que dans chacune des demi-coques, chacun des éléments déformables est retenu d'un côté par la présence sur leur partie 32 du plateau 14 et de l'autre côté par la présence du bossage présentant les surfaces 28 en extrémité de chacune des languettes 30, car chacune des surfaces 28 est apte à venir en contact avec la surface 56 de l'orifice 18. D'autre part, quand le plateau 14 vient en contact avec la face arrière 60 d'une demi-coque, la surface 28 de chacune des languettes 30 est éloignée de la surface 56 de la demi-coque. Inversement, lorsque chacune des surfaces 28 de chacune des languettes 30 est en contact avec la surface 56 de la demi-coque, le plateau 14 n'est plus en contact avec la surface 60 de la demi-coque. Enfin, pour l'utilisation, l'organe allongé 2 est ensuite inséré dans l'ensemble ainsi monté.

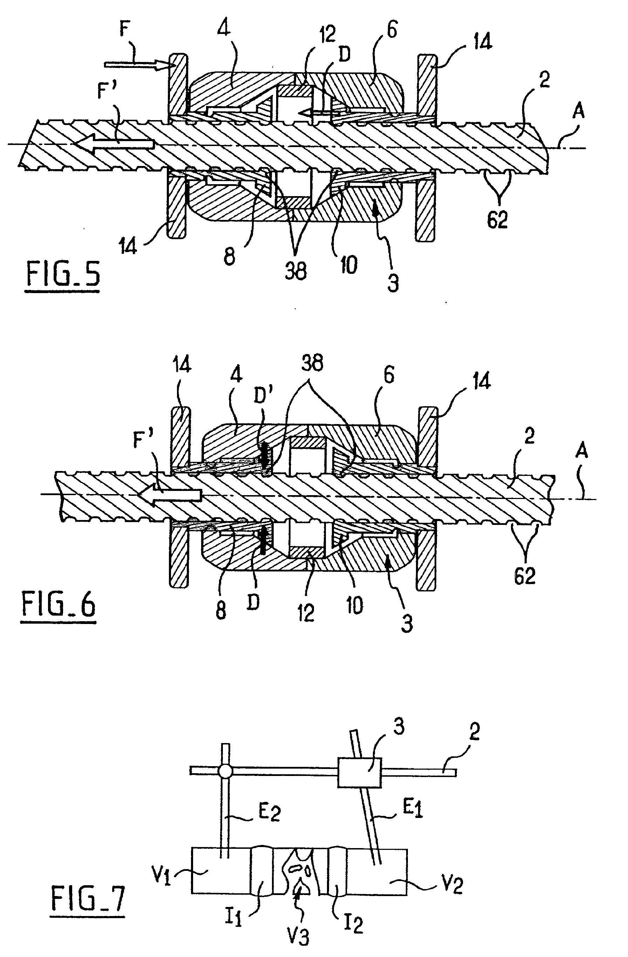

Nous allons maintenant décrire le fonctionnement du système de réglage en position 1 en référence aux figures 5, 6 et 7. Une colonne vertébrale comporte une vertèbre V3 présentant une fracture multiple. Cette vertèbre V3 est liée à deux vertèbres saines V1 et V2 de part et d'autre par des disques intervertébraux I1 et I2. On suppose que l'organe allongé 2 est lié à un élément E2 d'un instrument de chirurgie en prise avec la vertèbre V1, alors que le corps 3 est lié à un second élément E1 de ce même instrument de chirurgie en prise avec la vertèbre V2. Sur la figure 5, on va décrire le fonctionnement pour déplacer l'organe allongé 2 dans le sens de la flèche F'. Pour cela, l'opérateur applique un effort F sur le plateau 14 relié à l'élément déformable 8. Cet effort F permet de mettre en mouvement l'élément déformable 8 pour amener en contact le plateau 14 avec la face 60 de la demi-coque 4 du corps 3, les surfaces 28 des lames 30 n'étant plus en contact avec la surface 56 de la demi-coque correspondante. Les bossages circulaires 38 sont en prise dans une rainure 62 de l'organe allongé 2. L'autre élément déformable 10 a ses bossages 38 en prise dans une autre rainure 62 de l'organe allongé 2. Lors de l'application de la force F sur le premier plateau 14, les surfaces 28 de chacune des lames du second élément déformable 10 sont venus en contact avec la surface 56 de la demi-coque 6. De ce fait, le second plateau 14 lié au second élément déformable 10 s'est quant à lui éloigné de la face 60 de la seconde demi-coque 6. Ensuite, l'organe allongé 2 est déplacé selon le sens de la flèche F'. Du fait de la circularité des bossages 38 et des rainures 62 et du fait de l'ouverture vers l'extérieur des différentes languettes 30 qui se déforment au niveau de la rainure 36 essentiellement, les bossages 38 du premier élément déformable 8 vont s'échapper de la rainure 62, libérant ainsi l'organe allongé 2 de l'élément déformable 8. Dans le même temps, l'organe allongé 2 va entraîner avec lui le second élément déformable 10 selon un mouvement de direction D. Les surfaces 28 ne sont plus alors en contact avec la surface 56 de la demi-coque 6. Cet entraînement s'arrêtera lorsque le second plateau 14 viendra en contact avec la face 60 de la demi-coque 6. Pour les mêmes raisons que pour le premier élément déformable 8, l'organe allongé 2 va se dégager de la même manière du second élément déformable 10 du fait de la circularité des bossages 38 et des rainures 62. L'opérateur peut alors faire coulisser l'organe allongé 2 par rapport au corps 3 selon le sens de la flèche F' sur une longueur quelconque.

Une fois le déplacement de l'organe allongé 2 désiré est atteint, l'opérateur relâche son effort F qu'il exerçait sur le premier plateau 14. Les lames 30 de l'élément déformable 8 vont se refermer dès que les bossages 38 s'inséreront dans la première rainure 62 qui se présentera. Le mouvement dans le sens de la flèche F' de l'organe allongé 2 se poursuivant, l'organe allongé 2 entraîne avec lui l'élément déformable 8 jusqu'à ce que les faces 28 de chacune des languettes 30 de l'élément déformable 8 viennent en contact avec la surface 56 de la demi-coque 4 bloquant l'ensemble en position, en maintenant les bossages 38 dans la rainure 62 sous l'effort D' engendré par le contact de la surface 56 sur les surfaces 28.

De manière symétrique, en agissant sur le second plateau 14 de manière identique que précédemment, l'opérateur peut déplacer l'organe allongé 2 dans le sens opposé de la flèche F'.

De même, en agissant sur les deux plateaux 14 de manière simultanée comme décrit ci-dessus, l'opérateur peut alors faire coulisser selon les deux sens le corps 3 sur une longueur quelconque le long de l'organe allongé 2 afin de positionner le corps 3 à un emplacement quelconque désiré.

On pourra bien entendu apporter de nombreuses modifications à l'invention sans pour autant sortir du cadre de celle-ci.

Par exemple, l'organe allongé 2 pourra présenter en lieu et place des rainures 62 une surface moletée ou lisse. Dans ce cas, les éléments déformables pourront présenter en place et lieu des bossages 38 une surface moletée. Le blocage en position se fera alors par friction.

## Revendications

1. Système de réglage en position pour un instrument de chirurgie, notamment pour la colonne vertébrale, comportant un organe allongé (2) apte à être relié à un premier élément de l'instrument, et un corps (3) apte à être fixé à l'organe allongé et relié à un deuxième élément de l'instrument, **caractérisé en ce qu'**il comprend des moyens de liaison (8,10) de l'organe allongé (2) au corps(3) agencés pour avoir un état déverrouillé dans lequel ils autorisent un coulissement (F') de l'organe allongé (2) par rapport au corps (3) et un état verrouillé dans lequel ils interdisent le coulissement de l'organe allongé par rapport au corps.

2. Système de réglage en position selon la revendication 1, **caractérisé en ce que** les moyens de liaison sont agencés pour interdire le coulissement (F') de l'organe allongé par rapport au corps par effet de coin.

3. Système de réglage en position selon la revendication 1 ou 2, **caractérisé en ce que** les moyens de liaison comprennent au moins un élément déformable de manière élastique (8,10).

4. Système de réglage en position selon les revendications 2 et 3, **caractérisé en ce que** l'élément déformable est apte à réaliser l'effet de coin.

5. Système de réglage en position selon l'une des revendications 2 à 4, **caractérisé en ce que** le système est agencé de sorte que, dans l'état verrouillé, le coulissement est interdit selon un seul sens de déplacement.

6. Système de réglage en position selon l'une des revendications 3 à 5, **caractérisé en ce que**, l'élément déformable de manière élastique présentant une surface d'appui (38), l'organe allongé présente une face de contact (62) apte venir en contact avec la surface d'appui, lors de l'état verrouillé.

7. Système de réglage en position selon la revendication 6, **caractérisé en ce que** la face de contact (62) est lisse, le blocage s'effectuant par friction.

8. Système de réglage en position selon la revendication 6, **caractérisé en ce que** la face de contact (62) est moletée.

9. Système de réglage en position selon l'une des revendications 6 à 8, **caractérisé en ce que** la surface d'appui (38) comporte un moletage.

10. Système de réglage en position selon l'une des revendications 6 à 9, **caractérisé en ce que** la face de contact présente des creux (62), notamment des rainures.

11. Système de réglage en position selon la revendication 10, **caractérisé en ce que** la surface d'appui comporte un bossage (38) s'étendant en saillie, apte à être reçu dans les creux.

12. Système de réglage en position selon l'une des revendications 3 à 11, **caractérisé en ce que** l'élément déformable est fendu sur toute ou partie de sa longueur.

13. Système de réglage en position selon la revendication 12, **caractérisé en ce que** l'élément déformable comporte des languettes déformables (30).

14. Système de réglage en position selon l'une des revendications 3 à 13, **caractérisé en ce que** l'élément déformable comporte une extrémité de mise en oeuvre de déverrouillage (32).

15. Système de réglage en position selon l'une des revendications 3 à 14, **caractérisé en ce que** l'élément déformable est de forme générale conique.

16. Système de réglage en position selon la revendication 14 ou 15, **caractérisé en ce que** l'extrémité de mise en oeuvre (32) comporte un rebord d'appui (14).

17. Système de réglage en position selon l'une des revendications 3 à 16, **caractérisé en ce que** les moyens de liaison comprennent un second élément déformable de manière élastique (10)

18. Système de réglage en position selon la revendication 17, **caractérisé en ce que** le second élément déformable est apte à venir interdire le coulissement selon l'autre sens de déplacement.

19. Système de réglage en position selon la revendication 17 ou 18, **caractérisé en ce que** le second élément déformable (10) est le symétrique en miroir du premier élément déformable (8).

20. Système de réglage en position selon l'une des revendications 1 à 19, **caractérisé en ce que** le corps (3) est démontable en au moins deux parties (4,6).

21. Système de réglage en position selon la revendication 20, **caractérisé en ce que** le corps comporte un moyen de liaison (12) des deux parties, notamment une bague.

22. Instrument de chirurgie orthopédique, notamment pour la chirurgie de la colonne vertébrale, **caractérisé en ce qu'**il comporte un système de réglage en position selon l'une quelconque des revendications précédentes.
